# EUROPEAN PATENT APPLICATION

(11) **EP 2 384 750 A1**
(43) Date of publication of application: **09.11.2011**
(21) Application number: 10181682.5
(22) Date of filing: 29.09.2003
(51) Int. Cl.: A61K 31/20, A61K 31/202, A61P 3/10

(54) **Improved Glycemic Control for Prediabetes and/or Diabetes Type II using Docohexaenoic Acid**

(30) Priority: 27.09.2002 US 413859 P
(62) Divisional of application: 03754939.1
(71) Applicant: MARTEK BIOSCIENCES CORPORATION, Columbia, MD 21045 (US)
(72) Inventor: Arterburn, Linda, Ellicott City, MD MD21043 (US); Benisek, Diane, Severna Park, MD MD21146 (US); Hoffman, James, Blue Bell, PA PA19422 (US); Oken, Harry, Columbia, MD MD21044 (US); Van Elswyk, Mary, Longmont, CO CO80503 (US)
(74) Representative: Wallis, Naomi Rachel

(57) **Abstract**

This invention is directed to methods for treating patients with metabolic syndrome, prediabetes and/or type II diabetes mellitus by administering docosahexanoic acid (DHA) alone or in combination with diabetes-related medicines.

## Description

### CROSS-REFERENCED APPLICATION

This application claims priority to U.S. Provisional application No. 60/413,859 filed on September 27, 2002 which is hereby incorporated by reference in its entirety.

### BACKGROUND

### Field of the Invention

This invention is directed to methods of treating patients with metabolic syndrome, prediabetes and/or Type II diabetes mellitus by administering docosahexaenoic acid (DHA) alone or in combination with diabetes-related medications.

### Review of Related Art

Type II Diabetes Mellitus (T2DM) is defined as a serious, chronic disorder characterized by impaired carbohydrate, protein and fat metabolism. Over time, diabetes can lead to blindness, limb loss, kidney failure, cardiovascular disease and early death. Insulin resistance (defined as the state of resistance to insulin-mediated glucose disposal and resulting compensatory hyperinsulinemia) is a characteristic of Type II Diabetes Mellitus (T2DM) that often precedes development of the disease.

Any intervention that can safely prevent or delay the onset of T2DM is of particular interest for a variety of medical and economic reasons. It is estimated that 16 million Americans are prediabetic and that 11% per year of those pre-diabetics convert to T2DM. The morbidity of T2DM (manifested by microvascular disease leading to diabetic glomerulosclerosis and end-stage renal disease, retinopathy causing blindness, and neuropathy and macrovascular disease causing accelerated atherosclerosis leading to coronary and cerebrovascular diseases such as heart attack, peripheral vascular disease and stroke) is both medically and fiscally devastating for patients. Lost productivity, high cost of medical care and mortality have a major economic impact in the workplace. Current pharmacological therapies of T2DM are increasingly reported to have characteristic side effects and resulting morbidity, such as lactic acidosis (50% fatal) and long-term 2.5-fold increase in cardiovascular (CV) mortality.

In the blood, glucose binds irreversibly to hemoglobin molecules within red blood cells. The amount of glucose that is bound to hemoglobin is directly tied to the concentration of glucose in the blood. Since red blood cells have a lifespan of approximately 90 days, measuring the amount of glucose bound to hemoglobin can provide an assessment of average blood sugar control during the 60 to 90 days prior to the test. This is the purpose of the gylcated hemoglobin tests, most commonly the hemoglobin Alc (HbAlc) measurement. Since the test results give feedback on the previous two to three months, getting an HbAlc test done every three months provides data on average blood sugars. For instance, the chart below shows the approximate relationship between HbAlc and average blood sugar values. Typically, 4 to 6% is considered normal.

| Alc(%) | Mean Plasma Glucose | |
|---|---|---|
| | mg/dl | mmol/l |
| 4 | 65 | 3.5 |
| 5 | 100 | 5.5 |
| 6 | 135 | 7.5 |
| 7 | 170 | 9.5 |
| 8 | 205 | 11.5 |
| 9 | 240 | 13.5 |
| 10 | 275 | 15.5 |
| 11 | 310 | 17.5 |
| 12 | 345 | 19.5 |

Studies on the effects of polyunsaturated fatty acids on glucose control in diabetic and prediabetic patients have to this point been inconclusive. Fish oil is a source of ω-3 polyunsaturated fatty acids including both eicosapentaenoic acid (EPA, C20:5) and docosahexaenoic acid (DHA, C22:6). Fasching, et al., (1991, Diabetes 40(5):583-589) disclosed that fish oil did not impact fasting concentrations of glucose or insulin or induced glycemia and insulin response. Rivellese, et al, (1996, Diabetes Care 19(11):1207-13) showed that supplementation of subjects with impaired glucose control or Type 2 diabetes with 2-3 g of fish oil per day containing long-chain n-3 polyunsaturated fatty acid (PUFA) for 6 months did not alter serum insulin, fasting glucose, HbAlc levels or glucose tolerance tests. Stiefel et al., (1999, Ann Nutr Metab: 43(2):113-20) reported that administration of 330 mg DHA and 660 mg EPA per day resulted in a significant decrease in HbAlc levels in Type I diabetics. U.S. Patent 5,034,415 to Rubin (1991) reports a difference between naturally esterified fatty acids compared to the free fatty acid form in their effect on blood sugar levels. WO 02/11564 discusses nutritional supplements which may include lipid sources to be incorporated into the diet of diabetics. However, Friedberg, C.E. (1998 Diabetes Care 21(4):494-500) conducted a meta-analysis of 26 trials reported in the literature concerned with fish oil and diabetes. The analysis revealed that fish oil ingestion is associated with decrease in serum triglycerides and increase in LDL cholesterol, but without significant effect on HbAlc. Blood glucose showed borderline significant increases in Type II patients, which in the analysis appeared to be associated with DHA rather than EPA. Based on this meta-analysis of 26 trials, it would appear that fish oil could be useful for treating dyslipidemia in diabetics, but not for affecting glucose metabolism. Another recent meta-analysis of fish oil supplementation in T2DM by Montori et al., (2000 Diabetes Care: 23(9): 1407-1415) showed no statistically significant effect of fish oil on glycemic control as measured by fasting blood glucose or HbAlc. The triglyceride lowering effect of fish oil in T2DM was confirmed.

Studies with fish oil which contains both EPA and DHA clearly cannot differentiate among effects due to EPA, effects due to DHA and effects that require both fatty acids. In a study by Shimura, et al. (1997 Biol. Pharm. Bull. 20(5):507-510) mice were dosed with DHA ethyl ester at 100 mg/kg body weight (e.g., 7 g/d for 70 kg man). This dose of DHA reduced blood glucose and plasma triglycerides and enhanced insulin sensitivity in obese diabetic mice, but not normal or lean diabetic mice. However, the KK-Ay mouse used by Shimura et al. is not reflective of the mechanism by which Type II diabetes develops in humans. The KK-Ay mouse is genetically obese and therefore develops Type II diabetes almost immediately after birth. In contrast, Type II diabetes in humans is obesity- and age-related, typically developing after the age of 50 following at least a decade of impaired glucose tolerance and/or insulin insensitivity. A more appropriate mouse model, the NSY mouse, has become available. The NSY mouse develops Type II diabetes later in life following a disruption of the glucose/insulin metabolic response (Ueda et al., 2000, Diabetologia; 43(7):932-938). This more appropriate model has not been used in studies like those reported by Shimura, et al. In any case, the extremely high dose of fatty acid used in the Shimura study would be difficult and impractical for human therapy.

### SUMMARY OF INVENTION

It is an object of this invention to provide a novel method for improved glucose control in prediabetic and Type II diabetic patents.

It is another object of this invention to provide safer antidiabetic agents or combinations of agents than those employed in current standard of care, which combination will enhance glycemic control while reducing associated drug side-effects. In a more preferred embodiment, the combination of agents provide enhanced glycemic control while contributing a side effect profile akin to placebo. These and other objectives are met by one or more of the following embodiments.

In one embodiment, this invention provides a method for improving glucose control by administering DHA in an amount sufficient to reduce the percentage of glycosylated hemoglobin while minimizing or eliminating the side effects of the fatty acid (belching, bloating, abdominal distress and other GI symptoms) and the impracticality and/or expense of very high dose DHA, especially if such high dose DHA were given as fish oil or fish oil derivative. This invention further provides a method for improving glucose control as measured by glycosylated hemoglobin (HbAlc) by administering DHA on a regular basis in an amount sufficient to clinically reduce HbAlc levels.

In a particular embodiment, this invention provides a method for treating prediabetic patients by administering at least about 1 g/day of DHA as triglyceride oil to patients with metabolic syndrome or patients with impaired glucose control (but not yet necessarily diagnosed with Type II diabetes) as measured by elevated fasting glucose levels (about 110 to about 127 mg/dL) and/or elevated fasting insulin levels (> about 6 µU/ml) or mild/early type II diabetes. The patients would ingest DHA chronically with the goal of delaying the onset of Type II diabetes and/or maintaining better glucose control.

In another particular embodiment, this invention provides a method for eliminating or reducing patient exposure to Rx pharmaceutical (abbreviation for prescribed medication) currently prescribed for T2DM and increasingly prescribed for prediabetics with strong family histories and otherwise considered at high risk of developing T2DM. The ability of DHA to improve glucose control by moderating insulin insensitivity can lessen, delay or perhaps even eliminate the need for such Rx therapies as metformin (e.g., GLUCOPHAGE) with its potential for a devastating array of adverse events in a significant percentage of recipients. This invention provides a method of reducing the dosage of Rx pharmaceuticals given to patients to control glucose (or to moderate sensitivity/insensitivity and/or secretion of insulin) or otherwise to treat T2DM by co-administration of DHA with such Rx pharmaceutical. Because DHA contributes to improved glucose control there is a lessening (or even eliminating) of the dosage requirement of the Rx pharmaceutical, resulting in a lessening of patient exposure to the side effects of the Rx pharmaceutical. The compositions and methods of the invention have minimal side effects particularly when compared to the Rx pharmaceutical used alone. Unwanted side effects may include constipation, renal toxicity, gastro-intestinal ulcerations and/or bleeding.

One embodiment provides a method for improving glucose control in a patient through the administration of docosahexaenoic acid to the patient in an amount sufficient to reduce fasting blood glucose in the patient. Another embodiment provides a method for improving glucose control as measured by glycosylated hemoglobin (HbAlc) in blood from a patient comprising administering DHA to the patient on a periodic basis in an amount sufficient to reduce the portion of circulating hemoglobin that is glycosylated.

Another embodiment provides a method for treating diabetes comprising administering to an individual in need thereof an effective amount of DHA and a second pharmaceutical. Preferably, the second pharmaceutical is an antidiabetic. More preferably, the antidiabetic is insulin, a sufonylurea, an alpha-glucosidase inhibitor, a biguanide, a meglitinide, a thiazolidinedione or a combination or mixture thereof. In another preferred embodiment the antidiabetic is administered substantially contemporaneously with the DHA. In another embodiment, when a hyopglycemic agent and/or antidiabetic agent is administered, it may be administered in a dose less than the dose required to control blood glucose in the absence of DHA administration.

For each of the recited embodiments, it is also possible to measure or assess the level status of the patient and/or improvement in glucose regulation, impaired glucose control and/or insulin regulation. In another embodiment, the onset of Type II diabetes mellitus is delayed. In another embodiment, glucose control as measured by Frequently Sampled Intravenous Glucose Tolerance Testing (FSIGT) is improved. In another embodiment, the glucose control is improved according to HbAlc determination. In another embodiment, the blood HbAlc is reduced compared to a patient which has not received DHA.

In one embodiment the patient is prediabetic. In one embodiment, the patient exhibits fasting glucose between about 1,10 to about 127 mg/dL. In another embodiment, the patient exhibits fasting insulin greater that 6 µU/ml. In another embodiment, the patient exhibits triglyceride/HDL-C ratio of greater than 3. In another embodiment, the patient exhibits blood HbAlc greater than about 7%. In another embodiment, the patient exhibits at least three symptoms selected from abdominal obesity, high triglycerides, low HDL cholesterol, high blood pressure and fasting glucose greater than 100 mg/dL. In one embodiment, the relative amount of glycosylated hemoglobin is reduced without inducing side effects of excessive fatty acid dosing.

In another embodiment, the patient may exhibit one or more of the following: fasting glucose between about 110 to about 127 mg/dL; fasting insulin greater than about 6 µU/ml; a triglyceride/HDL-C ratio of greater than about 3; HbAlc blood greater than about 7%, wherein one or more are improved upon administration of DHA as compared to a patient which has not received DHA. In another embodiment administration of the compositions of the invention results in a patient with delayed onset of Type II diabetes mellitus who may exhibit improved glucose control as measured by FSIGT as compared to a patient which has not received DHA.

Another embodiment provides a method for treating diabetes comprising administering more than about 500 mg of DHA to an individual with a HbAcl greater than about 5% over a twenty-four hour period wherein a reduced amount of an antidiabetic agent is administered during the same twenty-four hour period to provide a reduced HbAcl or fasting insulin compared to a patient who receives the same amount of antidiabetic agent but has not been administered DHA. In another embodiment, the patient is protected against peripheral artery disease associated with both early type II and pre-type II diabetes.

Embodiments of the invention will provide methods for reducing the costs and/or side effects associated with taking antidiabetic medications when compared to patients who have not been administered DHA. The cost benefits include reduced fees for medications, reduced office fees associated with visits, and fewer testing fees (such as a reduced number of kidney or liver work ups) as a result of taking lower doses of antidiabetics compared to a patient who has not been administered DHA. Similarly, the side effects associated with antidiabetics will be reduced as lower doses may be administered in conjunction with the DHA to achieve similar glucose regulation as compared to sole administration of the antidiabetic.

In a clinical study in which DHA-containing single cell oil (DHASCO) capsules were co-administered with statin medication to patients with dyslipidemia, it was noted that HbAlc or glycosylated hemoglobin levels (a marker for glycemic control) were reduced in a clinically relevant manner in the high dose group (1000 mg DHA/day) after one year of treatment, when compared to the low dose group (200 mg DHA per day). Thus, the present inventors have discovered that DHA has a long term effect (as shown by reduction in glycosylated hemoglobin levels reflecting longer term glucose control integrated over 2-3 months). Finally, the inventors have discovered that therapy using DHA-containing oils can be effective at DHA levels that are not excessive (*e.g*., at levels which minimize side effects associated with fatty acid ingestion).

### BRIEF DESCRIPTION OF THE FIGURE

The figure shows long-term glycemic control as measured by glycosylated hemoglobin (HbAlc) in dyslipidemic patients treated with either 200 mg/day or 1000 mg/day of docosahexaenoic acid for 12 months.

### DETAILED DESCRIPTION OF THE EMBODIMENTS

Administration of DHA is effective in improving glycemic control in patients that may have metabolic syndrome with an increased risk of developing Type II diabetes. Metabolic syndrome is a constellation of lipid and non-lipid risk factors of metabolic origin. Metabolic syndrome is diagnosed when three or more of the following risk factors are present: abdominal obesity (men >40" waist, women >35"), high triglycerides (≥150 mg/dL), low HDL cholesterol (men <40 mg/dL women<50 mg/dL), high blood pressure (≥130/≥85), and high fasting glucose (≥110 mg/dL). Metabolic syndrome is often accompanied by increased levels of C-reactive protein (*i.e*., levels between about 3 mg/L and about 10 mgL). Small LDL particle size is also characteristic of this syndrome. The estimated prevalence of metabolic syndrome in the US population is 24% and up to 42% for persons between 60 and 69 years of age. (Metabolic syndrome is also called "Syndrome X" or the "Insulin Resistance Syndrome [IRS]). JAMA 2001;285:2846-2897. Moderate to high doses (greater than 200 mg DHA per day) may provide improved glucose control, by a mechanism in which DHA lowers mean blood glucose.

### Target Patient Population

Patients who may benefit from therapy according to this invention include prediabetic patients. These may be patients with metabolic syndrome. In particular, it is preferred to treat patients with impaired glucose control as determined by a fasting glucose greater than about 127 mg/dL, or even patients with fasting glucose greater than about 110 mg/dL. An alternative criterion for suitable patients is fasting insulin greater than 6 µU/ml. Another criterion for suitable patients is elevated triglyceride/HDL-C ratio, especially a weight ratio of at least about 4.6. Another criterion for suitable patients is a determination of a genetic predisposition to type II diabetes mellitus (*e.g*., family history, genetic susceptibility based on ethnicity, identification of susceptibility through gene screening or linkage analysis). This invention may also be used to treat hypertensive patients, recognizing that in addition to its demonstrated ability to reduce blood pressure (Mori et al., 1999 Hypertension. 34:253-260), as many as 50% of hypertensives go on to develop metabolic syndrome and/or type II diabetes. This invention may also be used to treat patients with systemic low-grade inflammation, particularly patients with elevated C-reactive protein, an acute phase reactant associated with systemic and local inflammation (CRP), in excess of 3.9 mg/L (measured as described in Hafner, et al., 2002). Therapy according to this invention may also be sued for patients who have been diagnosed with T2DM; these patients will particularly benefit from the combination therapy disclosed herein.

### Therapeutic Compositions

Suitable patients are treated according to this invention by chronic administration of a therapeutic composition containing DHA. For each of the recited embodiments, the DHA may be administered from any number of sources and in varying amounts of purity. Preferably, the DHA is administered as an oil which substantially comprises DHA. In a more preferred embodiment, the DHA is a microbial oil with greater than 10% DHA, more preferably greater than 15% DHA, and more preferably greater than 20% DHA while preferably being substantially free of other PUFAs. In the above embodiments, DHA may be administered as a free fatty acid or ethyl ester thereof. Preferably, DHA is administered in a composition which contains no other PUFA, or which contains no other ω-3 PUFA greater than 4% of total fatty acid, or more preferably no greater than 3%, or more preferably no greater than 2% of total fatty acid, or more preferably no greater than 1% of total fatty acid, or administered in the absence of eicosapentaenoic acid (EPA). In another embodiment, DHA is administered in a composition which has an EPA content less than one-fifth that of DHA. In another embodiment, DHA is administered in a food product, which preferably contains less than one-fifth as much EPA as DHA. In another preferred embodiment, DHA is administered in a triglyceride oil which contains no other long chain PUFA, which are meant to be PUFAs with C:20 or longer chains.

Preferably the DHA will be in the form of an oil for easier assimilation. (Triglycerides are a conventional source for dietary fatty acids.) More preferably, the oil will be substantially free of other ω-3 PUFA, in particular, no ω-3 PUFA other than DHA equal to 4% or more of the total fatty acid (TFA) content, or more preferably 3% or more, or more preferably 2% or more, and most preferably 1% or more. Even more preferably, the oil will be substantially free of EPA (e.g., <4% of total fatty acid, or more preferably <3%, or more preferably <2%, and most preferably <1%).

In one embodiment, DHA may be administered as a triglyceride oil containing at least 70% DHA, more preferably at least 75%, more preferably more than 80%, more preferably at least 85%, more preferably at least 90%, more preferably more than 95%, more preferably greater than 99%. To obtain a composition containing at least 70% of the fatty acids as DHA, one can subject a DHA-containing oil (e.g., a single cell oil from an algal source, such as Thraustochytriales or dinoflagellates) to hydrolysis and esterification to produce fatty acid monoesters, especially ethyl or methyl esters. The fatty acid esters are then subjected to known purification techniques, such as urea complexation, distillation, molecular distillation/fractionation, chromatography, etc., to recover a fraction with at least 70% DHA. The fractionated DHA mono esters, preferably C₁-C₄ alkyl chains, may be administered in that form, or the DHA may be transesterified to glycerol esters for administration or the esters may be hydrolyzed to provide free fatty acids for administration. C₁-C₄ alkyl groups may be either substituted (e.g. with hydroxyl, chloro, bromo, fluoro and iodo), unsubstituted, branched or unbranched. Non-limiting examples include methyl, ethyl, propyl, butyl.

Although the DHA-containing oils can be administered to patients alone, more commonly, they will be combined with one or more pharmaceutically acceptable carriers and, optionally, other therapeutic ingredients. Acceptable carriers are those which are compatible with the other components of the formulation and not deleterious to the patient. It will be appreciated that the preferred formulation can vary with the condition and age of the patient.

The fatty acids may be from any source including, natural or synthetic oils, fats, waxes or combinations thereof. Moreover, the fatty acids may be derived from non-hydrogenated oils, partially hydrogenated oils or combinations thereof. Non-limiting exemplary sources of fatty acids include seed oil, fish or marine oil, canola oil, vegetable oil, safflower oil, sunflower oil, nasturtium seed oil, mustard seed oil, olive oil, sesame oil, soybean oil, corn oil, peanut oil, cottonseed oil, rice bran oil, babassu nut oil, palm oil, low erucic rapeseed oil, palm kernel oil, lupin oil, coconut oil, flaxseed oil, evening primrose oil, jojoba, tallow, beef tallow, butter, chicken fat, lard, dairy butterfat, shea butter or combinations thereof. Specific non-limiting exemplary fish or marine oil sources include shellfish oil, tuna oil, mackerel oil, salmon oil, menhaden, anchovy, herring, trout, sardines or combinations thereof. Preferably, the source of the fatty acids is fish or marine oil, soybean oil or flaxseed oil, or microbially produced oil.

Particularly preferred oils are produced by microbial fermentation, as described in U.S. Patent Nos. 5,492,938 and 5,130,242, or International Patent Publication No. WO 94/28913, each of which is incorporated herein by reference in its entirety.

It is also possible for the dosage form to combine any forms of release known to persons of ordinary skill in the art. These include immediate release, extended release, pulse release, variable release, controlled release, timed release, sustained release, delayed release, long acting, and combinations thereof. The ability to obtain immediate release, extended release, pulse release, variable release, controlled release, timed release, sustained release, delayed release, long acting characteristics and combinations thereof is known in the art.

Any biologically-acceptable dosage form known to persons of ordinary skill in the art, and combinations thereof, are contemplated. Examples of such dosage forms include, without limitation, chewable tablets, quick dissolve tablets, effervescent tablets, reconstitutable powders, elixirs, liquids, solutions, suspensions, emulsions, tablets, multi-layer tablets, bi-layer tablets, capsules, soft gelatin capsules, hard gelatin capsules, caplets, lozenges, chewable lozenges, beads, powders, granules, particles, microparticles, dispersible granules, cachets, douches, suppositories, creams, topicals, inhalants, aerosol inhalants, patches, particle inhalants, implants, depot implants, ingestibles, injectables (including subcutaneous, intramuscular, intravenous, and intradermal), infusions, health bars, confections, animal feeds, cereals, yogurts, cereal coatings, foods, nutritive foods, functional foods and combinations thereof. Most preferably the compositions and methods of the invention utilize a form suitable for oral administration.

Formulations of the present invention suitable for oral administration can be presented as discrete units, such as capsules or tablets, each of which contains a predetermined amount of DHA oil or a predetermined amount of a suitable combination of DHA oils. These oral formulations also can comprise a solution or a suspension in an aqueous liquid or a non-aqueous liquid. The formulation can be an emulsion, such as an oil-in-water liquid emulsion or a water-in-oil liquid emulsion. The oils can be administered by adding the purified and sterilized liquids to a prepared enteral formula, which is then placed in the feeding tube of a patient who is unable to swallow.

Soft gel or soft gelatin capsules may be prepared, for example by dispersing the formulation in an appropriate vehicle (vegetable oils are commonly used) to form a high viscosity mixture. This mixture is then encapsulated with a gelatin based film using technology and machinery known to those in the soft gel industry. The industrial units so formed are then dried to constant weight.

In one preferred embodiment, the DHA microbial oil is incorporated into gel capsules. It will be recognized that any known means of producing gel capsules can be used in accordance with the present invention. Compressed tablets can be prepared by, for example, mixing the microbial oil(s) with dry inert ingredients such as carboxymethyl cellulose and compressing or molding in a suitable machine. The tablets optionally can be coated or scored and can be formulated so as to provide slow or controlled release of the pharmaceuticals therein. Other formulations include lozenges comprising DHA oil in a flavored base, usually sucrose and acacia or tragacanth.

Chewable tablets, for example may be prepared by mixing the formulations with excipients designed to form a relatively soft, flavored, tablet dosage form that is intended to be chewed rather than swallowed. Conventional tablet machinery and procedures, that is both direct compression and granulation, i.e., or slugging, before compression, can be utilized. Those individuals involved in pharmaceutical solid dosage form production are versed in the processes and the machinery used as the chewable dosage form is a very common dosage form in the pharmaceutical industry.

Film coated tablets, for example may be prepared by coating tablets using techniques such as rotating pan coating methods or air suspension methods to deposit a contiguous film layer on a tablet.

Compressed tablets, for example may be prepared by mixing the formulation with excipients intended to add binding qualities to disintegration qualities. The mixture is either directly compressed or granulated then compressed using methods and machinery known to those in the industry. The resultant compressed tablet dosage units are then packaged according to market need, i.e., unit dose, rolls, bulk bottles, blister packs, etc.

The invention also contemplates the use of biologically-acceptable carriers which may be prepared from a wide range of materials. Without being limited thereto, such materials include diluents, binders and adhesives, lubricants, plasticizers, disintegrants, colorants, bulking substances, flavorings, sweeteners and miscellaneous materials such as buffers and adsorbents in order to prepare a particular medicated composition.

Binders may be selected from a wide range of materials such as hydroxypropylmethylcellulose, ethylcellulose, or other suitable cellulose derivatives, povidone, acrylic and methacrylic acid co-polymers, pharmaceutical glaze, gums, milk derivatives, such as whey, starches, and derivatives, as well as other conventional binders known to persons skilled in the art. Exemplary non-limiting solvents are water, ethanol, isopropyl alcohol, methylene chloride or mixtures and combinations thereof. Exemplary non-limiting bulking substances include sugar, lactose, gelatin, starch, and silicon dioxide.

The plasticizers used in the dissolution modifying system are preferably previously dissolved in an organic solvent and added in solution form. Preferred plasticizers may be selected from the group consisting of diethyl phthalate, diethyl sebacate, triethyl citrate, cronotic acid, propylene glycol, butyl phthalate, dibutyl sebacate, castor oil and mixtures thereof, without limitation. As is evident, the plasticizers may be hydrophobic as well as hydrophilic in nature. Water-insoluble hydrophobic substances, such as diethyl phthalate, diethyl sebacate and castor oil are used to delay the release of water-soluble vitamins, such as vitamin B6 and vitamin C. In contrast, hydrophilic plasticizers are used when water-insoluble vitamins are employed which aid in dissolving the encapsulated film, making channels in the surface, which aid in nutritional composition release.

Compositions of the invention may be administered in a partial, i.e., fractional dose, one or more times during a given period, a single dose during a given period of time, a double dose during a given period of time, or more than a double dose during a given period of time (e.g. 24 or 48 hour periods). Fractional, double or other multiple doses may be taken simultaneously or at different times during the given period. The doses may be uneven doses with regard to one another or with regard to the individual components at different administration times.

Formulations suitable for topical administration to the skin can be presented as ointments, creams and gels comprising the DHA oil in a pharmaceutically acceptable carrier. A preferred topical delivery system is a transdermal patch containing the oil to be administered. In formulations suitable for nasal administration, the carrier is a liquid, such as those used in a conventional nasal spray or nasal drops.

Formulations suitable for parenteral administration include aqueous and non-aqueous sterile injection solutions which optionally can contain antioxidants, buffers, bacteriostats and solutes which render the formulation isotonic with the blood of the intended recipient; and aqueous and non-aqueous sterile suspensions which can include suspending agents and thickening agents. The formulations can be presented in unit-dose or multi-dose containers. A preferred embodiment of the present invention includes incorporation of the DHA oil into a formulation for providing parenteral nutrition to a patient.

The microbial oil compositions of the present invention need not be administered as a pharmaceutical composition. They also can be formulated as a dietary supplement, such as a vitamin capsule or as food replacement in the normal diet. The microbial oils can be administered as a cooking oil replacement formulated so that in normal usage the patient would receive amounts of DHA sufficient to elevate the concentrations of this fatty acid in the blood and in membranes of affected patients. A special emulsion type margarine could also be formulated to replace butter or ordinary margarine in the diet. The single cell microbial oils could be added to processed foods to provide an improved source of DHA. The oil can be microencapsulated using gelatin, casein, or other suitable proteins using methods known in the art, thereby providing a dry ingredient form of the oil for food processing.

It should be understood that in addition to the ingredients particularly mentioned above, the formulations of this invention can include other suitable agents such as flavoring agents, preservatives and antioxidants. In particular, it is desirable to mix the microbial oils with an antioxidant to prevent oxidation of the DHA. Such antioxidants would be food acceptable and could include vitamin E, carotene, BHT or other antioxidants known to those of skill in the art.

### Therapeutic Protocols

For each of the recited embodiments of the invention, DHA administration is preferably chronic. In another embodiment, the DHA is administered in an amount greater than 200 mg/day, more preferably greater than 400 mg/day, more preferably greater than 600 mg/day, more preferably greater than 800 mg/day, more preferably greater than 1000 mg/day, more preferably greater than 1,100 mg/day, more preferably greater than 1,200 mg/day, more preferably greater than 1,500 mg/day. In another embodiment the amount of DHA is preferably less than 7 g/day, more preferably less than 6 g/day, more preferably less than 5 g/day, most preferably less than 4g/day. Intervening dosages, such as 300 mg/day, 400 mg/day, 500 mg/day, are also contemplated by the invention and the invention expressly contemplates any dosage greater than 200 mg/day, in 1 mg/day increments (e.g., 201 mg/day, 202 mg/day, 203 mg/day...301 mg/day, 302 mg/day, ...etc.).

Typically DHA will be administered to the patient in accordance with any embodiment of this invention on a periodic basis (*i.e*. chronically or episodically) in an amount greater than 200 mg/day, preferably at least 600 mg/day, more preferable at least 1000 mg DHA per day, even more preferably greater than 1.1 g DHA per day, while minimizing or eliminating side effects of excessive fatty acid dosing, such as belching, bloating, abdominal distress and other GI symptoms. In view of the side effects resulting from excess fatty acid administration, very high dose ω-3 fatty acid dosing is impractical as well as expensive, especially if fish oil is used as a source of DHA. Thus, the dose of DHA is preferably less than 7 g/day; more preferably less than 6 g/day; even more preferably less than 5 g/day. DHA will typically be administered periodic basis, such as for at least 3 months, 6 months, or at least one year, more preferably for two or more years, or for five or ten years or even for life. In one embodiment, the DHA is administered as a triglyceride oil, preferably containing at least 70% DHA, or a triglyceride oil which contains no other ω-3 PUFA greater than 2% of total fatty acid. Preferably the DHA is administered in the absence of eicosapentaenoic acid (EPA) or in a triglyceride oil which has an EPA content less than one-fifth that of DHA, preferably in a food product that contains less than one-fifth as much EPA as DHA.

Typically DHA will be administered in a high dose (greater than 200 mg/day), preferably at least 600 mg/day, more preferably greater than 800 mg/day, more preferably at least 1 g/day, more preferably greater than 1.1 g/day, more preferably greater than 1.2 g/day, more preferably greater than 1.3 g/day, more preferably greater than 1.4 g/day, or more preferably greater than 1.5 g/day while minimizing or eliminating side effects of excessive fatty acid dosing, such as belching, bloating, abdominal distress and other GI symptoms. In view of the side effects resulting from excess fatty acid administration, very high dose ω-3 fatty acid dosing is impractical as well as expensive, especially if fish oil is used as a source of DHA. Thus, the dose of DHA is preferably less than 7 g/day; more preferably less than 6 g/day; even more preferably less than 5 g/day. Amounts of DHA as described herein are expressed as the weight of DHA methyl ester equivalent to the DHA content of the dosage form. DHA may also be administered in conjunction with an anti-platelet agent, such as aspirin. DHA will be administered chronically, typically for at least 6 months, or at least one year, more preferably for two or more years, or for five or ten years or even for life.

One suitable therapeutic regimen would be to administer approximately 1000 mg of DHA daily as DHASCO (i.e., DHA-containing single cell oil) capsules to patients with metabolic syndrome or patients with poor glucose control (but not yet necessarily diagnosed with Type II diabetes) as measured by elevated fasting glucose levels (110-127 mg/dL) and/or elevated fasting insulin levels (>6 µU/ml). The patients would continue to take DHA chronically with the goal of delaying the onset of Type II diabetes and maintaining better glucose control.

In accordance with this invention, administration of DHA as described herein will delay onset of Type II diabetes mellitus. Therapy according to this invention may also delay onset of metabolic syndrome. Therapy according to this invention may also protect against peripheral artery disease in both early type II or pre-type II diabetes. Effectiveness of therapy according to this invention may also be detected by intermediate measurement of improved glucose control (as measured by, e.g., FSIGT), or improved glucose control detected by reduced blood (or plasma) HbAlc at or below 7%. For the purposes of this invention protection against a disease or disease state such as coronary artery disease, cerebrovascular disease or peripheral artery disease is meant to include a reduction in the risk for the disease, a delay in disease onset, or a need for a reduced medical routine including doctor visits and/or medication dosages or frequency. Further, protection against a disease also includes the prevention or amelioration of at least one symptom associated with the disease or disease state. Effectiveness of therapy according to this invention may also be detected by intermediate measurement of improved insulin sensitivity (as measured by, e.g., FSIGT), or improved glucose control detected by reduced blood HbAlc at or below 7%. Therapy according to this invention may also protect against peripheral artery disease in both early type II or pre-type II diabetes.

The dose of DHA for a particular patient can be determined by the skilled clinician using standard pharmacological approaches in view of the above factors. The response to treatment may be monitored by analysis of blood or body fluids in the patient. The skilled clinician will adjust the dose and duration of therapy based on the response to treatment revealed by these measurements.

### Combination Therapy

DHA may be used alone, but in particularly preferred embodiments, it is administered concurrently with one or more other therapeutic agents. The concurrent agents may be directed at the same symptomatic or causative effects, or at different therapeutic targets. "Concurrent administration of two agents" as used herein means that both agents are present in pharmacologically effective levels in the circulation at the same time. Concurrent administration may be achieved by formulating both agents in the same composition, but it may also be achieved by simultaneous ingestion of doses of each agent or by administration of the two agents sequentially, so long as pharmacological effectiveness is achieved. Combination packaging described below with indicia for concurrent administration is contemplated by this invention.

Substantially contemporaneously means delivery of a second pharmaceutical, preferably an antidiabetic, within twenty-four hours of delivery of a DHA dosage of the invention. More preferably the second pharmaceutical is delivered within 12 hours, more preferably 6 hours, and more preferably 1 hour of delivery of the second pharmaceutical. In another embodiment, it is preferred that a DHA dosage is provided within 1 hour of delivery of the second pharmaceutical, more preferably 45 minutes, more preferably 30 minutes, and most preferably within 15 minutes of delivery of the second pharmaceutical.

Likewise, the compositions of the invention may be provided in a blister pack or other such pharmaceutical package. Further, the compositions of the present inventive subject matter may further include or be accompanied by indicia allowing individuals to identify the compositions as products for glucose regulation. The indicia may further additionally include an indication of the above specified time periods for administering the compositions. For example the indicia may be time indicia indicating a specific or general time of day for administration of the composition, or the indicia may be a day indicia indicating a day of the week for administration of the composition. The blister pack or other combination package may also include a second pharmaceutical product, e.g. a typical diabetes medication which should be taken in addition to the compositions of the invention.

In a particular embodiment, this invention provides a method for treating hypertensive and/or prediabetic patients by concurrent administration of at least 1 g/day of DHA, preferably as triglyceride oil, and at least one Rx antidiabetic agent to T2DM patients, or patients with metabolic syndrome and/or patients with impaired glucose control (but not yet necessarily diagnosed with Type II diabetes) as measured by elevated fasting glucose levels (110-127 mg/dl) and/or elevated fasting insulin levels (>6 µU/ml) and essential hypertension (blood pressure equal to or greater than 140/90 mmHg). Rx antidiabetic agents include oral antidiabetics such as chromium picolinate, first or second generation sulfonylureas, biguanides, thiazolidinediones and/or alpha-glucosidase inhibitors. Concurrent administration of DHA and one or more of the oral antidiabetic agents will provide for enhanced glucose control with lower doses of the antidiabetic agents than would be possible without concurrent administration of DHA. A typical therapeutic protocol would consist of daily administration of 400 mg to 2 g of DHA as a triglyceride oil and blood glucose levels would be monitored starting within at least three months after initiating DHA dosing. One or more antidiabetic agents would be administered at a dose that achieved the desired blood glucose level (typically <110 mg/dL). The antidiabetic dose and/or the DHA dose should be adjusted periodically consistent with normal clinical practice. Again, the DHA may be administered as fractionated DHA mono esters, preferably C₁-C₄ alkyl chains as described above, the DHA may be transesterified to glycerol esters for administration, or as the free fatty acid.

Antidiabetic drugs are commonly subdivided into six groups: insulin, sufonylureas, alpha-glucosidase inhibitors, biguanides, meglitinides, and thiazolidinediones. In addition to the above categories diabetes related medications may also include various supplements and vitamin routines. The below list is meant to be non-limiting, additionally, following the description of each commonly used antidiabetic is a list of common precautions/side effect which may be reduced or eliminate following the use of a decreased dosage as a result of the combination therapies described herein. In addition to the various common side effect there are numerous drug interactions for the below drugs, whereas DHA has fewer side effects, many of which are reversible. The sulfonylureas have a particularly long list of drug interactions, several of which may be severe. For example, the listing for the biguanide GLUCOPHAGE in the Physicians' Desk Reference, Medical Economics Company, the following are possible drug interactions, side effects, and instances when GLUCOPHAGE should be discontinued: side effects include lactic acidosis, renal function impairment, impaired heptatic function, and a decrease in vitamin B12 levels; instances when GLUCOPHAGE should be discotinued include hypoxic states, for surgical procedures, for alcohol intake, when nursing, and when pregnant; drug interactions include furosemide, nifedipine, and cationic drugs. For a full list of possible side effects see the GLUCOPHAGE Rx.

Insulin (Humulin, Novolin) is the hormone responsible for glucose utilization. It is effective in both types of diabetes, since, even in insulin resistance, some sensitivity remains and the condition can be treated with larger doses of insulin. Most insulins are now produced by recombinant DNA techniques, and are chemically identical to natural human insulin. Isophane insulin suspension, insulin zinc suspension, and other formulations are intended to extend the duration of action of insulin, and permit glucose control over longer periods of time. The greatest short term risk of insulin is hypoglycemia, which may be the result of either a direct overdose or an imbalance between insulin injection and level of exercise and diet. This may also occur in the presence of other conditions which reduce the glucose load, such as illness with vomiting and diarrhea. Treatment is with glucose in the form of glucose tablets or liquid, although severe cases may require intravenous therapy. Allergic reactions and skin reactions may also occur. Insulin is classified as category B in pregnancy, and is considered the drug of choice for glucose control during pregnancy. Insulin glargine (Lantus), an insulin analog which is suitable for once-daily dosing, is classified as category C, because there have been reported changes in the hearts of newborns in animal studies of this drug. The reports are essentially anecdotal, and no cause and effect relationship has been determined. Insulin is not recommended during breast feeding because either low of high doses of insulin may inhibit milk production. (Insulin administered orally is destroyed in the GI tract, and represents no risk to the newborn.)

Sulfonylureas (chlorpropamide [Diabinese], tolazamide [Tolinase], glipizide [Glucotrol] and others) which act by increasing insulin release from the beta cells of the pancreas. Glimepiride (Amaryl), a member of this class, also appears to have a useful secondary action in increasing insulin sensitivity in peripheral cells. All sulfonylurea drugs may cause hypoglycemia. Most patients become resistant to these drugs over time, and may require either dose adjustments or a switch to insulin. The list of adverse reactions is extensive, and includes central nervous system problems and skin reactions, among others. Hematologic reactions, although rare, may be severe and include aplastic anemia and hemolytic anemia. The administration of oral hypoglycemic drugs has been associated with increased cardiovascular mortality as compared with treatment with diet alone or diet plus insulin. The sulfonylureas are classified as category C during pregnancy, based on animal studies, although glyburide has not shown any harm to the fetus and is classified as category B. Because there may be significant alterations in blood glucose levels during pregnancy, it is recommended that patients be switch to insulin. These drugs have not been fully studied during breast feeding, but it is recommended that because their presence in breast milk might cause hypoglycemia in the newborn, breastfeeding be avoided while taking sulfonylureas.

Others examples include α-glucosidase inhibitors (acarbose [Precose], miglitol [Glyset]) which do not enhance insulin secretion but inhibit the conversion of disaccharides and complex carbohydrates to glucose. While this does not prevent conversion it does provide a delay and thus reduces the peak blood glucose levels. Alpha-glucosidase inhibitors are useful for either monotherapy or in combination therapy with sulfonylureas or other hypoglycemic agents. Alpha-glucosidase inhibitors are generally well tolerated, and do not cause hypoglycemia. The most common adverse effects are gastrointestinal problems, including flatulence, diarrhea, and abdominal pain. These drugs are classified as category B in pregnancy. Although there is no evidence that the drugs are harmful to the fetus, it is important that rigid blood glucose control be maintained during pregnancy, and pregnant women should be switched to insulin. Alpha-glucosidase inhibitors may be excreted in small amounts in breast milk, and it is recommended that the drugs not be administered to nursing mothers.

Metformin (Glucophage) is the only available member of the biguanide class. Metformin decreases hepatic glucose production, decreases intestinal absorption of glucose and increases peripheral glucose uptake and utilization. Metformin may be used as monotherapy, or in combination therapy with a sulfonylurea. Metformin causes gastrointestinal reactions in about a third of patients. A rare, but very serious, reaction to metformin is lactic acidosis, which is fatal in about 50% of cases. Lactic acidosis occurs in patients with multiple medical problems, including renal insufficiency. The risk may be reduced with careful renal monitoring, and careful dose adjustments to metformin. Metformin is category B during pregnancy. There have been no carefully controlled studies of the drug during pregnancy, but there is no evidence of fetal harm from animal studies. It is important that rigid blood glucose control be maintained during pregnancy, and pregnant women should be switched to insulin. Animal studies show that metformin is excreted in milk. It is recommended that metformin not be administered to nursing mothers.

Additionally, two members of the meglitinide class: repaglinide (Prandin) and nateglitinide (Starlix) act to stimulate insulin production. This activity is both dose dependent and dependent on the presence of glucose, so that the drugs have reduced effectiveness in the presence of low blood glucose levels. The meglitinides may be used alone, or in combination with metformin. Meglitinides are generally well tolerated, with an adverse event profile similar to placebo. The drugs are classified as category C during pregnancy, based on fetal abnormalities in rabbits given about 40 times the normal human dose. It is important that rigid blood glucose control be maintained during pregnancy, and pregnant women should be switched to insulin. It is not known whether the meglitinides are excreted in human milk, but it is recommended that these drugs not be given to nursing mothers.

Rosiglitazone (Avandia) and pioglitazone (Actos) are members of the thiazolidinedione class. They act by both reducing glucose production in the liver, and increasing insulin dependent glucose uptake in muscle cells and do not increase insulin production. These drugs may be used in combination with metoformin or a sulfonylurea. Thiazolidinediones are generally well tolerated, however they are structurally related to an earlier drug, troglitazone, which was associated with liver function problems. It is strongly recommended that all patients treated with pioglitazone or rosiglitazone have regular liver function monitoring. The drugs are classified as pregnancy category C, based on evidence of inhibition of fetal growth in rats given more than four times the normal human dose. It is important that rigid blood glucose control be maintained during pregnancy, and pregnant women should be switched to insulin. It is not known whether the thiazolidinediones are excreted in human milk, however they have been identified in the milk of lactating rats. It is recommended that these drugs not be administered to nursing mothers.

Formulation and dosing of antidiabetics is well-known and the method of this invention does not require any change except to reduce the dose to lower the pharmacologic level which achieves adequate glucose regulation in the presence of administered DHA.

### Examples

In order to facilitate a more complete understanding of the invention, Examples are provided below. However, the scope of the invention is not limited to specific embodiments disclosed in these Examples, which are for purposes of illustration only.

### Example 1

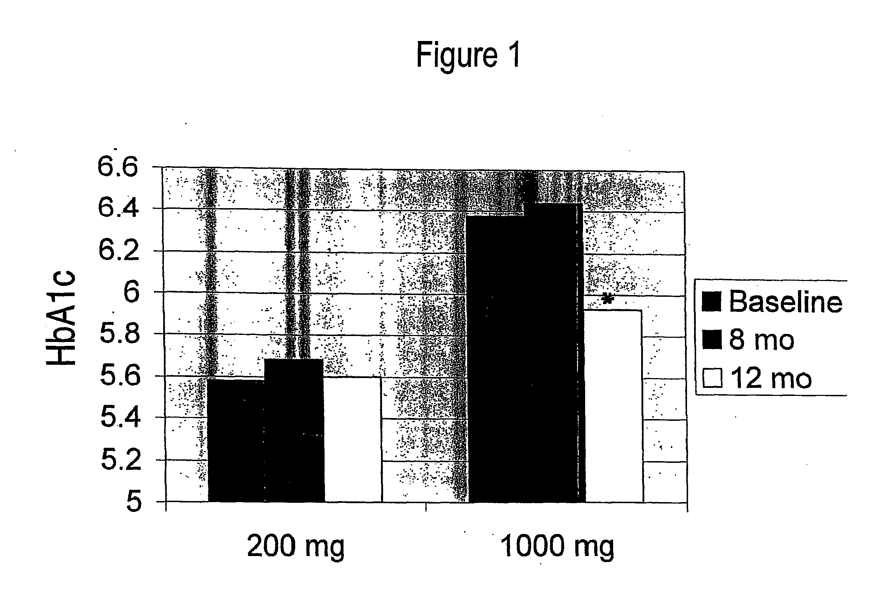

In a clinical study, DHASCO capsules (which contained DHA as a triglyceride oil extracted from *Crypthecodinium cohnii* cells, obtained from Martek Biosciences Corp., Columbia, Maryland) were co-administered with statin medication to patients with dyslipidemia. Hyperlipidemic patients already being treated with a stable dose of a statin medication but still failing to meet NCEP guidelines for LDL-cholesterol or triglycerides were treated with either 200 or 1000 mg of DHA daily for 12 months. HbAlc levels (glycosylated hemoglobin, a marker of glycemic control) were measured in plasma at baseline and after 8 or 12 months of treatment. The HBAlc levels were significantly reduced in the high dose group (1000 mg DHA/day) after one year of treatment compared to the low dose group (200 mg DHA per day). These results are shown in Figure 1.

In this study, thirteen of 20 patients treated with DHA showed reductions in CRP levels, for an overall reduction of 15%. Reduction in CRP of this extent is clinically significant, and may be correlated with a benefit of reduced risk of Type II diabetes onset, independent of other Type II risk factors.

### Example 2

DHASCO-S capsules (which contained DHA as a triglyceride oil extracted from *Schizochytrium* sp. cells, obtained from Martek Biosciences Corp., Columbia, Maryland) were used in the following study. Subjects (n=57) were enrolled in a randomized, double-blind, controlled trial to assess the response to 1.52 g of DHA per day for six weeks. Subjects were aged 21-80 and had HDL levels below the sex-specific median (a criterion for metabolic syndrome). The average triglyceride (TG) level at the beginning of the study was 169 - 179 mg/dl (metabolic syndrome criterion >150 mg/dL). The average distribution of LDL particles in this population was 44-50% small dense particles. Small dense particles are another lipid hallmark of metabolic syndrome. Taken together the subjects in this study exhibited up to 3 of the lipid markers of metabolic syndrome. The average waist circumference for men and women combined was about 100 +/- 2.5 cm (criterion for metabolic syndrome is about 88 cm for women and about 102 cm for men). The average blood pressure in the study was normotensive at 120/77 mmHg. In short, while this study did not specifically enroll subjects based on meeting criteria for metabolic syndrome, the majority presented with several lipid markers and a substantial number may also have met the waist circumference criterion.

The metabolic disturbance score was calculated to assess the relationship between independent variables and the criterion related to metabolic syndrome. The score is a composite of values for selected risk factors including: serum triglycerides, serum HDL cholesterol, systolic blood pressure, waist circumference, and fasting serum glucose. The following formula was utilized to calculate the metabolic score, where Z = the z score, i.e., the number of standard deviations above or below the mean value for each variable (Micciolo 1991; Maki 1994): Metabolic Disturbance Score = Z for triglycerides + Z for systolic blood pressure + Z for waist circumference + Z for glucose - Z for HDL cholesterol. Since triglyceride values were not normally distributed, a natural log-transformation was performed to normalize the distribution prior to calculating the Z score.

Additionally, pre-specified subgroup analyses were performed for serum lipid and metabolic disturbance score values. The triglyceride subgroups were split at the median baseline value (170 mg/dL). A Body Mass Index (BMI) of 30 kg/m² was chosen as the cut-point for BMI subgroups (BMI < 30 kg/m2 and BMI ≥30 kg/m²) because persons with a BMI ≥30 kg/m² are considered obese (Expert Panel 1998).

The results demonstrated a statistically suggestive (P=0.062) decrease in the change from baseline for the metabolic disturbance score with intake of the DHA capsules (-0.35) relative to the control (+0.32). In addition, there was a significant difference between treatments in the change from baseline metabolic disturbance scores for the subgroup of subjects with baseline triglycerides ≥170 mg/dL (-1.16 for the DHA treatment vs. 1.16 for the control; p = 0.001). Changes in TG (DHA - 25.4% change from baseline vs. -14.6% for control; p=0.001) and increases in HDL (DHA +9.0% vs. +5.3% for control; p=.102) contributed to the above results but individual results may have been achieved by changes in these alone or other metabolic syndrome criteria included in the assessment.

Finally, the distribution of LDL particles among the DHA consumers was significantly improved as compared to controls. Large LDL particles increased 32.4% in the DHA group from baseline to end of treatment while controls increased 5.4% (P value for control v. DHA; P=0.028). Small particles subsequently showed a decline among DHA consumers (-23.2% from baseline vs. -11.4% for controls; P =.191).

Metabolic syndrome is one of the underlying causes of T2DM (Grundy et al., 2002) and these results indicate that DHA consumption may mediate desirable change in up to 3 of the lipid markers of this syndrome.

### Example 3

The following clinical study may be carried out to validate the results of the clinical trial described in Example 1. For the purpose of the study a prediabetic will be an individual with a fasting glucose level between 100 mg/dL - 126 mg/dL. The study population of pre-diabetics will be randomly divided into three treatment groups comprising at least 100 individuals each. The first treatment group will receive DHA (as capsules containing DHA as a triglyceride oil extracted from *Crypthecodinium cohnii* cells, obtained from Martek Biosciences Corp., Columbia, Maryland) according to the invention in the amount of 1 g DHA/day. The second treatment group will receive EPA in the amount of 1 g EPA/day. The third treatment group will receive a placebo which will contain olive oil or a suitable substitute in the same amount of triglyceride. Each group will maintain the treatment course for a period of at least six months, more preferably one year. Over the evaluation period testing for fasting glucose will be performed monthly. Additionally, at least at the on set and conclusion of the study individuals will be assessed for their HbAlc levels. Upon completion the results may be expected to show that the DHA group has a maintained a higher number of prediabetic individuals (i.e. individuals with a blood glucose level below 127 mg/dL) as compared to the placebo group and the EPA group.

### Example 4

In another study, overweight, dyslipidemic adults will be randomized to receive placebo (corn/soy oil), 200 mg DHA or 1 g DHA from DHASCO for six months following a six-week run-in period. The patients must have elevated triglycerides and moderately low HDL cholesterol to qualify for the study. These patient will also typically be characterized as exhibiting abdominal obesity as well as three or more of the risk factors associated with metabolic syndrome and, as such are at increased risk of developing T2DM. Glucose control will be measured by assessing HbAlc levels and other risks factors associated with metabolic syndrome, including triglycerides, HDL cholesterol levels, as well as small LDL lipoprotein particle size, and blood pressure. These criteria will be measured at baseline and after three and six months of treatment. Upon completion, the results may be expected to show that the 1 g DHA group will exhibit improvement in the risk factors associated with metabolic syndrome as compared to baseline and the placebo group.

### Example 5

In another study, overweight or obese adults may be randomized to placebo or an appropriate dose of DHA from DHASCO for 4 months during an outpatient phase of the study. There is a strong correlation between abdominal obesity and the risk factors associated with metabolic syndrome. Fasting glucose, insulin, and HbAlc will be measured and an oral glucose tolerance test administered before and after treatment during 3-week inpatient phases of the study to test for clinically significant improvements in any of the outcomes associated with metabolic syndrome, including triglycerides, HDL and LDL cholesterol concentration, as well as, lipoprotein particle size, blood pressure, proinflammatory cytokines and prothrombic status. Upon completion the results may be expected to show that the DHA group will have improved glucose control and an improvement in the risk factors associated with metabolic syndrome as compared to baseline and the placebo group_{.}

### Example 6

In another study, subjects with metabolic syndrome who are at high risk of developing T2DM will be given an appropriate DHA dose (typically between 1 g and 3 g) or a placebo daily for a period of 1-2 years (depending on the number of subjects). The need for prescription drug treatment for T2DM will be determined by the following periodic tests: the HbAlc test, the Fasting Plasma Glucose (FPG), and the Oral Glucose Tolerance Test (OGTT). It may be expected that the number patients requiring prescription drug treatment in the DHA group will be fewer than in the placebo group.

### Example 7

In another study, subjects with T2DM on prescription drug treatment for the control of glucose metabolism will be randomized to receive either an appropriate dose of DHA or placebo daily. The dosage requirement for prescription drug treatment will be determined through the following periodic tests: the HbAlc test, the Fasting Plasma Glucose (FPG), and the Oral Glucose Tolerance Test (OGTT). This study will demonstrate the range and effectiveness of DHA administration on reducing/eliminating prescription drug needs for diabetic patients. The applicability of this study technique applies to all non-insulin classes of antidiabetic agents including Biguanides, Glucosidease inhibitors, Meglitinides, Sulfonylureas, and Thiazolidinediones.

Although the foregoing invention has been described in some detail by way of illustration and example for purposes of clarity of understanding, it will be obvious that certain changes and modifications may be practiced within the scope of the appended claims. Modifications of the above-described modes for carrying out the invention that are obvious to persons of skill in medicine, pharmacology, and/or related fields are intended to be within the scope of the following claims.

All publications and patent applications mentioned in this specification are indicative of the level of skill of those skilled in the art to which this invention pertains. All such publications and patent applications are herein incorporated by reference in their entirety to the same extent as if each individual publication or patent application was specifically and individually indicated to be incorporated by reference in its entirety.
1. A method for improving glucose control as measured by glycosylated hemoglobin (HbAlc) in blood from a patient comprising administering DHA to the patient on a periodic basis in an amount sufficient to reduce glycosylation levels of circulating hemoglobin the patient.
2. A method for treating diabetes comprising administering to an individual in need thereof an effective amount of DHA substantially contemporaneously with a second pharmaceutical.
3. The method of clause 1 wherein a second pharmaceutical is administered substantially contemporaneously with the DHA.
4. The method of clause 2 or 3 wherein the second pharmaceutical is an antidiabetic.
5. The method of clause 4, wherein the antidiabetic is insulin, a sufonylurea, an alpha-glucosidase inhibitor, a biguanide, a meglitinide, or a thiazolidinedione, or combinations thereof.
6. The method of clause 5 wherein a hypoglycemic agent is administered in a dose less than the dose required to control blood glucose in the absence of DHA administration.
7. The method of clause 4,5, or 6 further comprising a combination of two or more antidiabetics.
8. The method of clause 1 wherein the patient is prediabetic.
9. The method of clause 1 wherein onset of Type II diabetes mellitus is delayed.
10. The method of clause 1, wherein DHA is administered to a patient who exhibits fasting glucose between about 110 to about 127 mg/dL; fasting insulin greater that 6 µU/ml ; and a triglyceride/HDL-C ratio of greater than about 3; and/or HbAlc blood greater than about 7%; and said administration results in delayed onset of Type II diabetes mellitus; and glucose control as measured by FSIGT is improved and/or reduced blood HbAlc compared to a patient which has not received DHA.
11. The method of any preceding clause wherein the patient exhibits at least three symptoms selected from abdominal obesity, high triglycerides, low HDL cholesterol, high blood pressure and fasting glucose greater than 100 mg/dL.
12. The method of any preceding clause wherein the patient exhibits at least one of the following: fasting glucose between about 110 to about 127 mg/dL, fasting insulin greater than about 6 µU/ml, triglyceride/HDL-C ratio of greater than about 3, and a blood HbAlc greater than 7%.
13. The method of any preceding clause wherein glucose control as measured by FSIGT is improved.
14. The method of any preceding clause wherein glucose control is improved according to an HbAlc.
15. The method of any preceding clause wherein blood HbAlc is reduced compared to a patient which has not received DHA.
16. The method of any preceding clause wherein said patient is protected against peripheral artery disease associated with both early type II and pre-type II diabetes.
17. A method for treating diabetes comprising administering about 500 mg or more of DHA over a twenty-four hour period to an individual with a HbAcl greater than about 6% wherein a reduced amount of an antidiabetic is administered during the same twenty-four hour period to provide a reduced HbAcl or fasting insulin compared to a patient who has not been administered DHA.
18. The method of clauses 2 to 17, wherein side effects associated with taking an antidiabetic are reduced when compared to a patient who has not been administered DHA.
19. A method of treating an individual at risk of developing metabolic syndrome comprising:
   a) assessing an individual to determine if two or more risk factors are present wherein the risk factors are selected from abdominal obesity (men>40" waist, women>35"), high triglycerides (>150 mg/dL), low HDL cholesterol (men<40 mg/dL, women<50 mg/dL), high blood pressure (30/5), small LDL particle size and high fasting glucose (>110 mg/dL);
   b) providing said individual with a dosage of DHA which is greater than about 750 mg/day.
20. The method of any preceding clause wherein said administration of DHA is chronic.
21. The method of any preceding clause wherein the relative amount of glycosylated hemoglobin is reduced without inducing side effects of excessive fatty acid dosing.
22. The method of any preceding clause wherein DHA makes up at least about 70% of the fatty acids administered as a triglyceride oil, free fatty acids, fatty acid alkyl esters or combinations thereof.
23. The method of any preceding clause wherein DHA is administered in a triglyceride oil which contains no other ω3 PUFA greater than about 4% of total fatty acid.
24. The method of any preceding clause wherein DHA is administered in a triglyceride oil which has an EPA content less than about one-fifth that of DHA.
25. The method of any preceding clause wherein DHA is administered in a food product that contains DHA as a triglyceride oil, free fatty acids, fatty acid alkyl esters or combinations thereof.

## Claims

1. Use of a medicament for reducing HbAlc levels in a human individual suffering from diabetes comprising:
(a) measuring glycosylated haemoglobin (HbAlc) in the blood of the individual in need thereof; and
(b) administering an oral dosage form to the individual with a HbAlc greater than about 7% for at least one year wherein the dosage form comprises an oil having greater than about 20% DHA wherein the amount of the DHA administered is at least about 1 gram per day and wherein the DHA is in the form of a triglyceride oil or an ester and is substantially free of EPA.

2. Use of claim 1 wherein the DHA is administered substantially contemporaneously with a second pharmaceutical.

3. Use of claim 2 wherein the second pharmaceutical is an antidiabetic.

4. Use of claim 3 wherein the antidiabetic is insulin, a sufonylurea, an alpha-glucosidase inhibitor, a biguanide, a meglitinide, a thiazolidinedione, or combinations thereof.

5. Use of claim 4 wherein a hypoglycemic agent is for administration in a dose less than the dose required to control blood glucose in the absence of DHA administration.

6. Use of claim 4, further comprising a combination of two or more antidiabetics.

7. Use according to any preceding claim wherein the oil or formulation comprises >20% DHA of total fatty acid.

8. Use according to any preceding claim wherein the DHA is a triglyceride oil.

9. Use according to any preceding claim wherein the DHA is an ethyl ester.

10. Use according to any preceding claim wherein the oil or formulation comprises >70% DHA of total fatty acid.

11. Use according to any preceding claim wherein the DHA is administered 1 gram/day.
